# EUROPEAN PATENT APPLICATION

(11) **EP 3 771 438 A1**
(43) Date of publication of application: **03.02.2021**
(21) Application number: 20187635.6
(22) Date of filing: 24.07.2020
(51) Int. Cl.: A61B 17/122

(54) **TWO-PIECE LIGATION CLIP**

(30) Priority: 01.08.2019 US 201962881413 P; 10.01.2020 US 202062959297 P; 08.06.2020 US 202016895549
(71) Applicant: Covidien LP, Mansfield, MA 02048 (US)
(72) Inventor: BARIL, Jacob C., Norwalk, CT Connecticut 06851 (US); DININO, Matthew A., Newington, CT Connecticut 06111 (US); THOMAS, Justin, New Haven, CT Connecticut 06512 (US); PILLETERE, Roy J., North Haven, CT Connecticut 06473 (US); LAPIERRE, Nicolette R., Windsor Locks, CT Connecticut 06096 (US)
(74) Representative: Maschio & Soames IP Ltd

(57) **Abstract**

A ligation clip is formed from two separate beams that can be delivered to a surgical site for application to tissue independently of each other in an unstrained condition. The first and second beams can be subsequently coupled to each other and moved to a clamped position during application of the ligation clip to body tissue. In aspects of the disclosure, the first and second beams include latching structure to facilitate application of the ligation clip to tissues of different thicknesses. In other aspects of the disclosure, each of the pieces of the ligation clip can be a mirror image of the other piece of the ligation clip.

## Description

### CROSS-REFERENCE TO RELATED APPLICATIONS

This application claims the benefit of and priority to U.S. Provisional Patent Application Serial Nos. 62/959,297 filed January 10, 2020 and 62/881,413 filed August 1, 2019, the entire disclosures each of which are incorporated by reference herein.

### FIELD

This disclosure is generally related to ligation clips and, more particularly, to ligation clips formed from two pieces that are adjustable to accommodate tissues of variable thickness.

### BACKGROUND

Polymeric ligation clips typically include first and second beams that are coupled together at one end by a pivotable connection, e.g., living hinge, such that the first and second beams can be moved in relation to each other between open and clamped positions. The ligation clips can be applied to tissue endoscopically through a small diameter incision or through a small diameter cannula positioned through the incision to minimize trauma to a patient during a surgical procedure.

Typically, when polymeric clips are applied to tissue through a cannula and/or stored within an endoscopic clip applier, the clips are supported in a compressed or partially compressed state to minimize an overall dimension of the clips and facilitate delivery of the clips through the cannula or incision. Storing polymeric clips in a compressed or partially compressed state may impact the condition of the clips which may impact the performance of the clips. More specifically, storing the polymeric clips in a compressed or partially compressed state causes strain and/or material creep in the material of the polymeric clip, especially in the region of the living hinge, which may adversely impact the condition and/or performance of the polymeric clip.

### SUMMARY

In one aspect of the disclosure, a ligation clip includes a first beam and a second beam. The first beam has a first end portion, a second end portion, and a first clamping surface that is positioned between the first and second end portions. The first end portion of the first beam has a first finger and a second end portion of the first beam defines a first through bore. The first beam defines a first longitudinal axis. The second beam has a first end portion, a second end portion, and a second clamping surface that is positioned between the first and second end portions. The first end portion of the second beam has a second finger and the second end portion of the second beam defines a second through bore. The second beam defines a second longitudinal axis. The first finger of the first beam is received within the second through bore of the second beam and the second finger of the second beam is received within the first through bore of the first beam to secure the ligation clip in a clamped position.

In another aspect of the disclosure, a ligation clip includes a first beam defining a first longitudinal axis and a second beam defining a second longitudinal axis. The first beam has a first end portion, a second end portion, and a first clamping surface that is positioned between the first and second end portions. The first end portion of the first beam has a first finger and the second end portion of the first beam defines a first through bore. The second beam has a first end portion, a second end portion, and a second clamping surface that is positioned between the first and second end portions. The first end portion of the second beam has a second finger and the second end portion of the second beam defines a second through bore. The first finger of the first beam is received within the second through bore of the second beam and the second finger of the second beam is received within the first through bore of the first beam to secure the ligation clip in a clamped position. The first beam is a mirror image of the second beam.

In another aspect of the disclosure, a ligation clip includes a first beam defining a first longitudinal axis and a second beam defining a second longitudinal axis. The first beam has a first end portion, a second end portion, and a first clamping surface that is positioned between the first and second end portions. The first end portion of the first beam has a first finger and a second end portion of the first beam defines a first through bore. The second beam has a first end portion, a second end portion, and a second clamping surface that is positioned between the first and second end portions. The first end portion of the second beam has a second finger and the second end portion of the second beam defines a second through bore. The first and second fingers include a plurality of first teeth and the first and second through bores include a plurality of second teeth. The plurality of first teeth is engageable with the plurality of second teeth to retain the ligation clip in the clamped position. The first finger of the first beam is received within the second through bore of the second beam and the second finger of the second beam is received within the first through bore of the first beam to secure the ligation clip in a clamped position.

In aspects of the disclosure, the first and second fingers include first latching structure and the first and second through bores include second latching structure. The first latching structure is engagable with the second latching structure to retain the ligation clip in the clamped position.

In some aspects of the disclosure, the first and second clamping surfaces define a gap "G" in the clamped position, and the first latching structure of the first and second fingers is engageable with the first and second through bores in a plurality of relative positions to vary the size of the gap "G" in the clamped position of the ligation clip to accommodate tissues of different thicknesses.

In certain aspects of the disclosure, the first latching structure includes at least one first tooth formed on each of the first and second fingers and the second latching structure includes at least one second tooth formed on a wall defining each of the through bores. The at least one first tooth is engageable with the at least one second tooth to retain the ligation clip in the clamped position.

In aspects of the disclosure, the at least one first tooth includes a plurality of first teeth.

In some aspects of the disclosure, the at least one second tooth includes a plurality of second teeth.

In certain aspects of the disclosure, the first end portion of each of the first and second beams includes first posts that extend radially outward of the first and second clamping surfaces.

In aspects of the disclosure, the second end portion of each of the first and second beams includes second posts that extending radially outward of the first and second clamping surfaces.

In some aspects of the disclosure, the first and second beams are mirror images of each other.

In certain aspects of the disclosure, each of the at least one first tooth and each of the at least one second tooth includes a ramped surface and a lock surface. The lock surface defines a plane that is substantially parallel to the longitudinal axes of the first and second beams.

In aspects of the disclosure, the ramped surfaces of the at least one first tooth and the ramped surface of the at least one second tooth are positioned to engage each other as the ligation clip is moved towards the clamped position such that the at least one first tooth passes over the at least one second tooth in ratcheting fashion.

In some aspects of the disclosure, the lock surfaces of the at least one first tooth and the at least one second tooth are movable into engagement to retain the ligation clip in the clamped position.

In aspects of the disclosure, the first finger defines a first pair of spaced fingers and the second finger defines a second pair of spaced fingers, and each of the first and second pairs of spaced fingers defines a channel.

In some aspects of the disclosure, the first beam includes a first dividing wall that is positioned within the first through bore and the second beam includes a second dividing wall that is positioned within the second through bore, and the first dividing wall is received within the channel defined by the first pair of spaced fingers and the second dividing wall is received in the channel defined by the second pair of spaced fingers.

In certain aspects of the disclosure, the second latching structure is formed on walls defining the first and second through bores, and the first latching structure is formed on outwardly facing surfaces of the first and second pairs of spaced fingers.

In aspects of the disclosure, the second latching structure is formed on the first and second dividing walls, and the first latching structure is formed on inwardly facing surfaces of the first and second pairs of spaced fingers.

In aspects of the disclosure, each of the first and second fingers includes a wall defining a cavity, and the walls are flexible to allow wall the fingers to flex inwardly.

In aspects of the disclosure, each of the plurality of first teeth and each of the plurality of second teeth includes a ramped surface and a lock surface. The lock surfaces define planes that are substantially parallel to the first and second longitudinal axes of the respective first and second beams and the ramped surfaces of the plurality of first and second teeth are positioned to engage each other as the ligation clip is moved towards the clamped position such that the plurality of first teeth pass over the plurality of second teeth in ratcheting fashion.

In some aspects of the disclosure, the first end portion of each of the first and second beams includes first posts, and the second end portion of each of the first and second beams includes second posts. The first and second posts extend radially outward of the first and second clamping surfaces.

Other features of the disclosure will be appreciated from the following description.

### BRIEF DESCRIPTION OF THE DRAWINGS

Various aspects of the disclosed two-piece ligation clips are described herein below with reference to the drawings, wherein:
FIG. 1 is a side perspective view of the disclosed two-piece ligation clip with the pieces separated and positioned about a body vessel (shown in phantom) having a first diameter;
FIG. 2 is a cross-sectional view taken along section line 2-2 of FIG. 1;
FIG. 3 is a cross-sectional view of the two-piece ligation clip shown in FIG. 2 in the clamped position about the body vessel having the first diameter;
FIG. 4 is a cross-sectional view of the two-piece ligation clip shown in FIG. 1 with the pieces separated and positioned about a body vessel having a second larger diameter;
FIG. 5 is a side perspective view of the two-piece ligation clip shown in FIG. 1 with the ligation clip in a clamped position about the body vessel having the second larger diameter;
FIG. 6 is a side perspective view of the two-piece ligation clip shown in FIG. 5 in the clamped position about the larger diameter body vessel (shown in phantom);
FIG. 7 is a cross-sectional view taken along section line 7-7 of FIG. 6;
FIG. 8 is a side perspective view of another aspect of the disclosed two-piece ligation clip shown with the pieces separated and positioned about a body vessel (shown in phantom);
FIG. 9 is a cross-sectional view taken through the two-piece ligation clip of FIG. 8;
FIG. 10 is a cross-sectional view of the two-piece ligation clip shown in FIG. 9 in the clamped position about the body vessel having the first diameter;
FIG. 11 is a side perspective view of yet another aspect of the disclosed two-piece ligation clip shown with the pieces separated and positioned about a body vessel (shown in phantom);
FIG. 12 is a cross-sectional view taken through the two-piece ligation clip of FIG. 11;
FIG. 13 is a cross-sectional view of the two-piece ligation clip shown in FIG. 12 in the clamped position about the body vessel;
FIG. 14 is a side perspective view of yet another aspect of the disclosed two-piece ligation clip shown with the pieces separated and positioned about a body vessel (shown in phantom);
FIG. 15 is a cross-sectional view taken through the two-piece ligation clip of FIG. 14;
FIG. 16 is a cross-sectional view of the two-piece ligation clip shown in FIG. 15 in the clamped position about the body vessel;
FIG. 17 is a side perspective view of yet another aspect of the disclosed two-piece ligation clip shown with the pieces separated and positioned about a body vessel (shown in phantom);
FIG. 18 is a cross-sectional view taken through the two-piece ligation clip of FIG. 17; and
FIG. 19 is a cross-sectional view of the two-piece ligation clip shown in FIG. 18 in the clamped position about the body vessel.

### DETAILED DESCRIPTION

The disclosed two-piece ligation clips will now be described in detail with reference to the drawings in which like reference numerals designate identical or corresponding elements in each of the several views. However, it is to be understood that the disclosed aspects of the two piece ligation clips are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. In addition, directional terms such as upper, lower, top, bottom, and similar terms are used to assist in understanding the description and are not intended to limit the disclosure.

In this description, the term "endoscopic" is used generally to refer to endoscopic, laparoscopic, arthroscopic, and/or any other procedure conducted through a small diameter incision or cannula. Further, the term "clinician" is used generally to refer to medical personnel including doctors, nurses, and support personnel. Finally, the term "substantially" is used generally to refer to 90 percent to 110 percent of a referenced parameter.

The disclosed ligation clip is formed from two separate beams that can be delivered to a surgical site for application to tissue, e.g., a body vessel, independently of each other in an unstrained condition to minimize damage to the ligation clip during storage and/or delivery of the ligation clip to the surgical site. The first and second beams can be subsequently coupled to each other and moved to a clamped position during application of the ligation clip to body tissue. In aspects of the disclosure, the first and second beams include latching structure to facilitate application of the ligation clip to tissues of different thicknesses. In other aspects of the disclosure, each of the pieces of the ligation clip can be a mirror image of the other piece of the ligation clip to minimize manufacturing costs of the ligation clip.

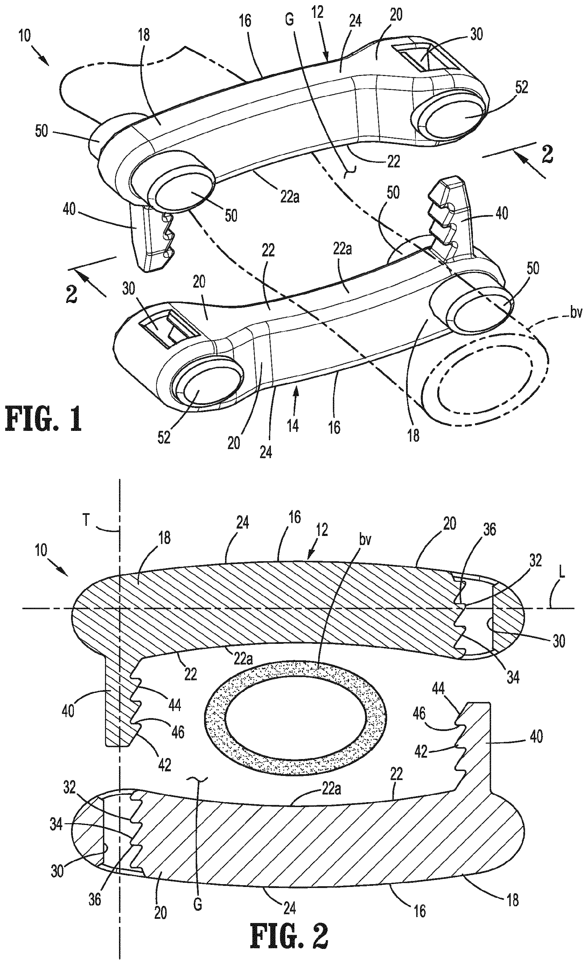

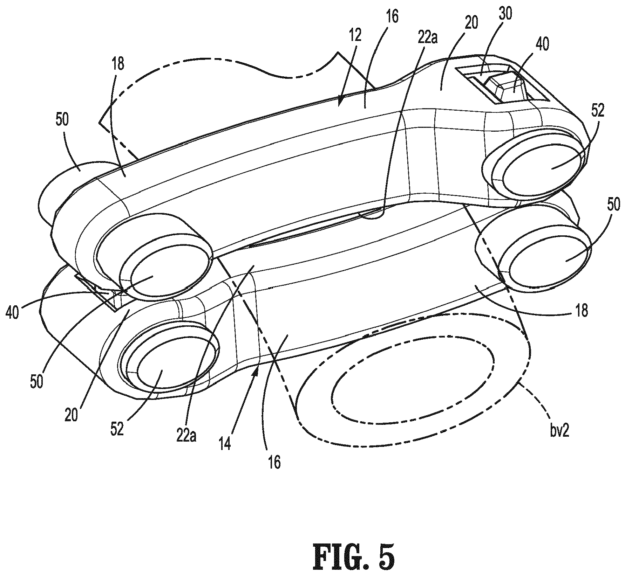

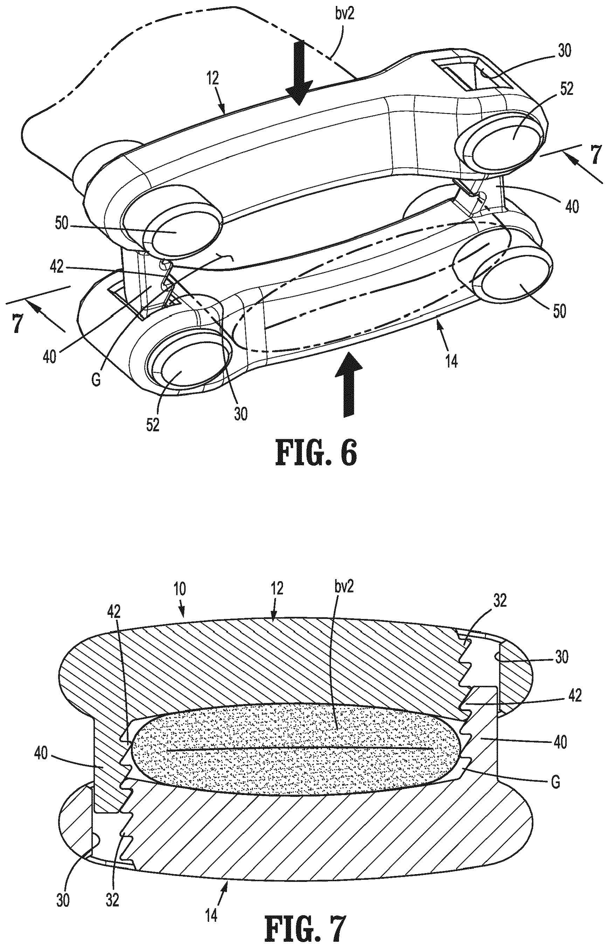

The disclosed two-piece ligation clip is shown generally in FIGS. 1-7 as ligation clip 10. FIGS. 1 and 2 illustrate the ligation clip 10 in an open position, i.e., a position in which the pieces of the ligation clip are spaced from each other to define a gap to receive tissue. The ligation clip 10 includes a first piece or beam 12 and a second piece or beam 14. In the open position, as defined above, the first and second beams 12, 14 are spaced from each other to define a gap "G" to receive tissue, e.g., body vessel "bv". In aspects of the disclosure, the beams 12, 14 are mirror images of each other.

Each of the first and second beams 12, 14 includes a body 16 that defines a longitudinal axis "L" (FIG. 2) and has a first end portion 18, a second end portion 20, an inner surface 22, and an outer surface 24. The first end portion 18 of each of the first and second beams 12, 14 defines a through bore 30 that extends from the inner surface 22 of the body 16 to the outer surface 24 of the body 16. In one aspect of the disclosure, the through bore 30 is defined by a wall having a plurality of teeth 32. In certain aspects of the disclosure, each of the teeth 32 has a ramped surface 34 and a lock surface 36 that defines a plane that is substantially parallel to the longitudinal axis "L" (FIG. 2) of the respective beam 12, 14. The second end 20 of the body 16 of each of the beams 12, 14 supports a finger 40 that defines an axis "T" (FIG. 2) that is substantially transverse to the longitudinal axis "L" of the respective beam 12, 14. The finger 40 and the through bore 30 form latching structure of the ligation clip 10 that functions to retain the ligation clip 10 in a clamped position about the body vessel "bv". In certain aspects of the disclosure, each of the fingers 40 includes at least one tooth 42 that sequentially engages the teeth 32 located on the wall defining the through bore 30 of a respective beam 12, 14 to secure the first and second beams 12, 14 together about tissue "bv" in several distinct positions. In each of the distinct positions, the gap "G" defined between the beams 12, 14 is different to accommodate tissues of different thicknesses. The at least one tooth 42 also has a ramped surface 44 and a lock surface 46 that defines a plane that is substantially parallel to the longitudinal axes "L" of the first and second beams 12, 14 when the ligation clip 10 is in a clamped position. Engagement between the locking surface 36 of the teeth 32 and the locking surface 46 of the tooth or teeth 42 prevents inadvertent separation of the first and second beams 12, 14 of the ligation clip 10. In certain aspects of the disclosure, the at least one tooth 40 includes a plurality of teeth 42 that are aligned along the axis "T" (FIG. 2).

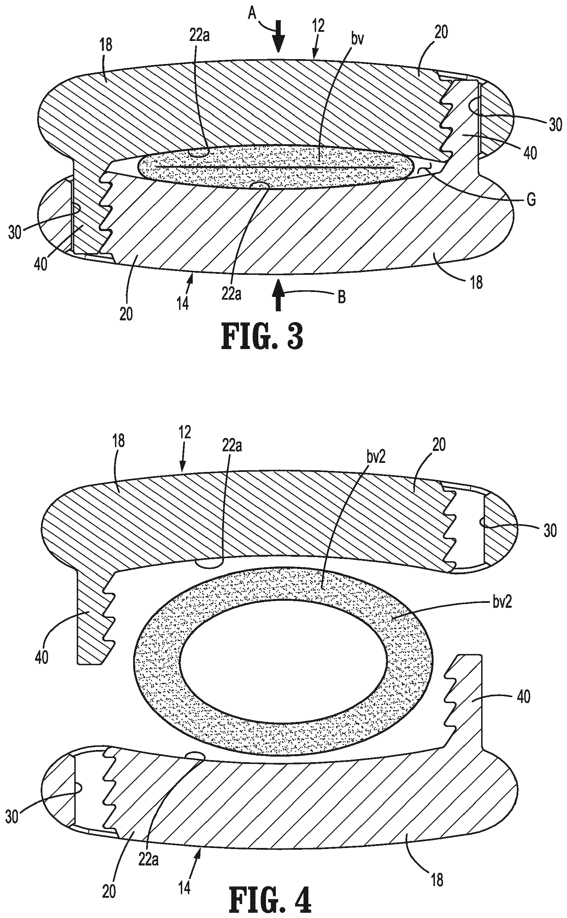

The inner surface 22 of the body 16 of each of the beams 12, 14 defines a clamping surface 22a that is located between the through bore 30 and the finger 40 of the respective beam 12, 14. The clamping surfaces 22a are positioned to engage tissue, e.g., the body vessel "bv", when the ligation clip 10 is moved to a clamped position (FIG. 3) the body vessel "bv". Although not shown, the clamping surfaces 22a of one or both of the beams 12, 14 may include retention structure to minimize any likelihood of slippage of the ligation clip 10 in relation to the body vessel "bv" when the ligation clip 10 (FIG. 3) is applied to the body vessel "bv". The retention structure can be in the form of protrusions or recesses of a variety of different configurations.

The first end portion 18 of the body 16 of the first and second beams 12, 14 includes posts 50 and the second end portion 20 of the body 16 of the first and second beams 12, 14 includes posts 52. The posts 50, 52 have a substantially oval shape and are configured to engage a track defined within a multi-fire clip applier (not shown) to sequentially guide a stack of ligation clips 10 into jaws of the clip applier.

FIG. 3 illustrates the ligation clip 10 as the ligation clip 10 is clamped about tissue, e.g., the body vessel "bv". In order to clamp or ligate a body vessel "bv", the beam 12 is positioned on one side of the body vessel "bv" and the beam 14 is positioned on an opposite side of the body vessel "bv". The beams 12, 14 are positioned with the clamping surfaces 22a of the first and second beams 12, 14 facing each other and the body vessel "bv". In addition, the first end portion 18 of the first beam 12 is positioned opposite to the second end portion 20 of the second beam 14, and vice versa. It is envisioned that the beams 12, 14 will be delivered to a surgical site by a clip applier (not shown) with the beams 12, 14 of the ligation clip 10 supported in spaced relation to each other in the clip applier. It is also envisioned that the beams 12, 14 will be aligned by and within the clip applier (not shown) prior to application to tissue. As stated above, one or both of the posts 50, 52 can be received within grooves or slots in the clip applier to support and or guide the ligation clips 10 within and along the clip applier.

When the first beam 12 and the second beam 14 are moved to their clamped position in the directions indicated by arrows "A" and "B" in FIG. 3, the body vessel "bv" is compressed between the clamping surfaces 22a of the first and second beams 12, 14 to ligate the body vessel "bv". When the first and second beams 12, 14 are moved towards the clamped position, the fingers 40 on the first end portion 18 of the first and second beams 12, 14 are received within the through bores 30 defined in the second end portions 20 of the first and second beams 12, 14. As the fingers 40 are received within the through bores 30, the teeth 42 on the fingers 40 sequentially engage the teeth 32 formed on the walls defining the through bores 30 to secure the fingers 40 within the through bores 30. When the teeth 42 engage the teeth 32, the ramped surfaces 34 of the teeth 32 engage the ramped surfaces 44 of the teeth 42 to allow the teeth 42 to pass over the teeth 32 and further into the through bore 30 in ratcheting fashion to clamp the body vessel "bv" between the clamping surfaces 22a of the first and second beams 12, 14. Each time a tooth 42 of a finger 40 passes over a tooth 32 within a respective bore 30, the size of the gap "G" defined between the clamping surfaces 22a of the first and second beams 12, 14 is reduced and the lock surfaces 46 of the teeth 42 engage the lock surfaces 36 of the teeth 42 to retain the finger 40 within the through bore 30. The configurations of the teeth 32 and 42 allow each of the beams 12, 14 to move in only one direction in relation to the other beam of the ligation clip 10, i.e., in the direction of arrows "A" and "B" (FIG. 3).

FIGS. 4-7 illustrate the ligation clip 10 clamped about relatively thicker tissue, e.g., body vessel "bv2", than that shown in FIGS. 1-3. Because the body vessel "bv2" is relatively thicker than tissue "bv" (FIG. 3), the finger 40 of each beam 12, 14 is received within the respective through bore 30 of the other of the beams 12, 14 to a lesser extent before the body vessel "bv2" is compressed between the clamping surfaces 22a of the beams 12, 14. As such, only forward most teeth 42 of the fingers 40 are engaged with teeth 32 positioned on the walls defining the through bores 30 when the tissue "bv2" is compressed. As such, a larger gap "G" is provided between the clamping surfaces 22a to accommodate the thicker tissue "bv2". It is envisioned that the length of the fingers 40 and/or the number of teeth 42 provided on the fingers 40 and/or within the through bores 30 may be selected to accommodate a larger range of tissue thicknesses.

Each of the beams 12, 14 of the ligation clip 10 may be formed at least in part of a resilient bioabsorbable and/or biocompatible polymeric material. Examples of suitable bioabsorbable and/or biocompatible polymeric materials include acetal polyoxymethylene (POM), polyethylene terephthalate (PET), polybutylene terephthalate (PBT), polyoxymethylene, polyetheretherketone (PEEK), polypropylene, and polyethylene or other thermoplastic materials having similar properties that can be injection molded. The ligation clip 10 may also be made at least in part of a polymeric material or materials in combination with radiolucent metal alloys. Alternately, other materials may be used to form the ligation clip 10 including biocompatible metals, plastics and composites.

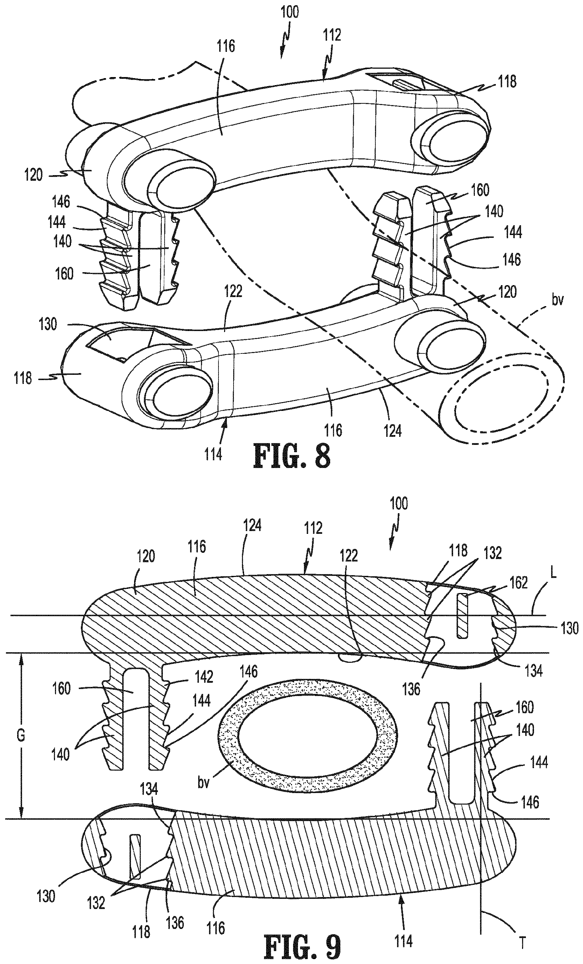

FIGS. 8-10 illustrate other aspects of the disclosed two-piece ligation clip shown generally as ligation clip 100. The ligation clip 100 is substantially identical to the ligation clip 10 (FIG. 1) but includes a modified latching structure. Accordingly, only the latching structure of the ligation clip 100 will be described in detail in this application.

The ligation clip 100 includes a first piece or beam 112 and a second piece or beam 114. As described above in regard to the ligation clip 10 (FIG. 1), the ligation clip 100 can move between open and clamped positions. In the open position illustrated in FIGS. 8 and 9, the first and second beams 112, 114 are spaced from each other to define a gap "G" (FIG. 9) to receive tissue, e.g., body vessel "bv". In certain aspects of the disclosure, the beams 112, 114 can be mirror images of each other, wherein each of the beams 112, 114 includes a body 116 that defines a longitudinal axis "L" (FIG. 9) and has a first end portion 118, a second end portion 120, an inner surface 122, and an outer surface 124. The first end portion 118 of each of the first and second beams 112, 114 defines a through bore 130 that extends from the inner surface 122 of the body 116 to the outer surface 124 of the body 116. In certain aspects of the disclosure, teeth 132 are spaced along walls defining the through bore 130. Each of the teeth 132 include a ramped surface 134 and a lock surface 136 that defines a plane that is substantially parallel to the longitudinal axis "L" (FIG. 2) of the respective beam 112, 114.

The second end 120 of the body 116 of each of the beams 112, 114 supports a pair of fingers 140. Each of the fingers 140 defines an axis "T" (FIG. 9) that is substantially transverse to the longitudinal axis "L" of the respective beam 112, 114. The fingers 140 are spaced from each other to define a channel 160 between the fingers 140. The fingers 140 and the through bore 130 form the latching structure of the ligation clip 100 and functions to retain the ligation clip 100 in one of a plurality of clamped positions about the body vessel "bv".

In aspects of the disclosure, the first end portion 118 of the beams 112, 114 includes a dividing wall 162 that is positioned within the channel 160. When the first and second beams 112, 114 are moved to one of the plurality of clamped positions, the fingers 140 area received within the through bore 130, the dividing wall 162 is received within the channel 160 between the fingers 140.

In certain aspects of the disclosure, each of the fingers 140 includes at least one outwardly facing tooth 142 that sequentially engages the teeth 132 located on the wall defining the through bore 130 of a respective beam 112, 114 to secure the first and second beams 112, 114 together about tissue "bv" in several distinct positions. In each of the distinct positions, the gap "G" defined between the beams 112, 114 is different to accommodate tissues of different thicknesses. The at least one tooth 142 also has a ramped surface 144 and a lock surface 146 that defines a plane that is substantially parallel to the longitudinal axes "L" of the first and second beams 112, 114 when the ligation clip 100 is in a clamped position. When the fingers 140 are inserted into the respective through bores 130 of the respective first and second beams 112, 114 to move the first and second beams 112, 114 to one of the clamped positions, the fingers 140 engage the wall that defines the through bore 130 and initially flex inwardly to allow the tooth or teeth 142 to pass over the respective teeth 132. As the tooth or teeth 142 pass over each of the respective teeth 132, the fingers 140 snap outwardly to move the tooth or teeth 142 into engagement with the teeth 132 to secure the first and second beams 112, 114 in one of the plurality of clamped positions. The ligation clip 100 operates in a substantially similar manner to the ligation clip 10. Accordingly, a description of the operation of the ligation clip 100 is not provided herein.

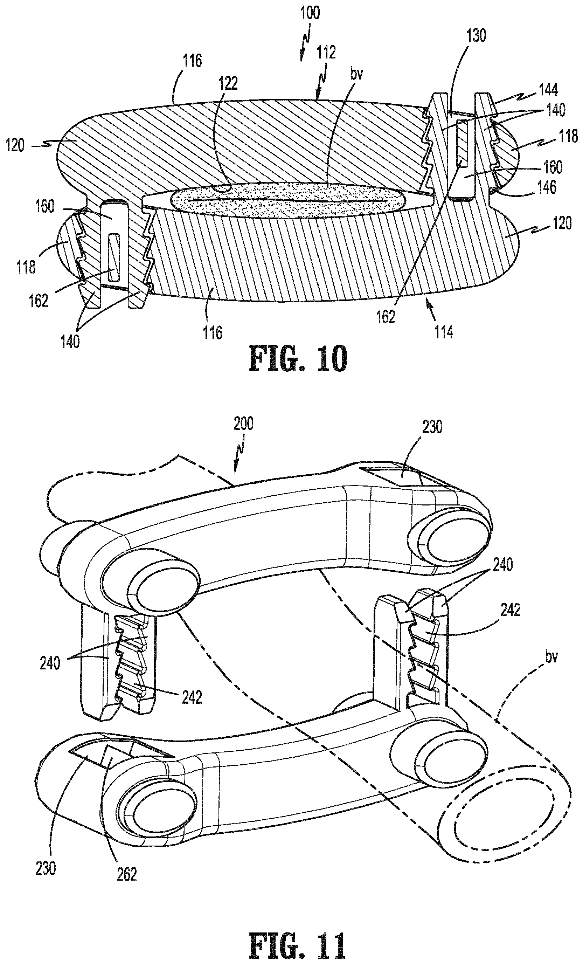

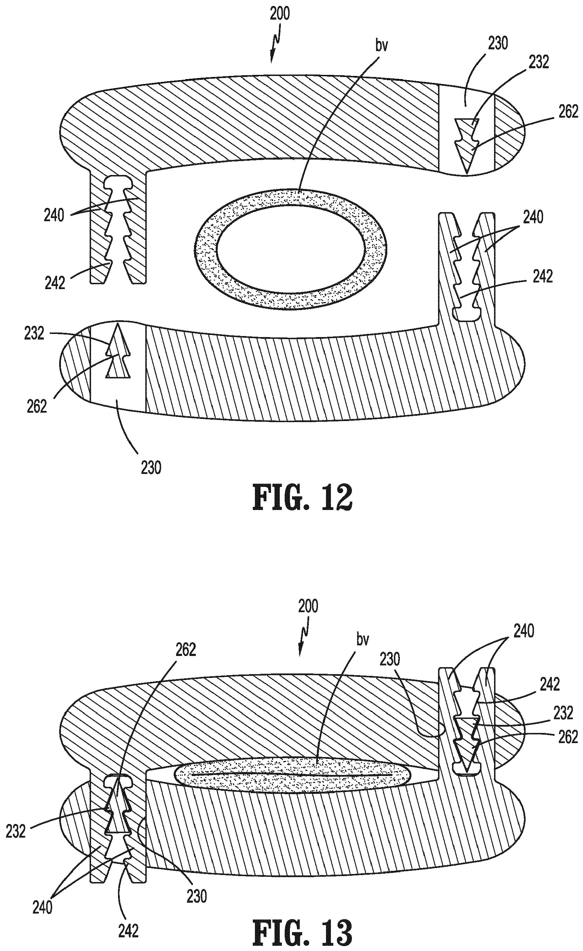

FIGS. 11-13 illustrate other aspects of the disclosed two-piece ligation clip shown generally as ligation clip 200. The ligation clip 200 is substantially identical to the ligation clip 100 (FIG. 8) but includes a modified latching structure. More specifically, in the ligation clip 200, the teeth 242 formed on the fingers 240 face inwardly towards each other and the teeth 232 are formed on the dividing wall 262 within the through bore 230. When the fingers 240 are inserted into the respective through bores 230 of the respective first and second beams 212, 214 to move the first and second beams 212, 214 to one of the clamped positions, the tooth or teeth 242 formed on the fingers 240 sequentially engage the teeth 232 on the dividing wall 262 within the through bore 230 and initially flex outwardly to allow the tooth or teeth 242 to pass over the respective teeth 232. As the tooth or teeth 242 pass over each of the respective teeth 232, the fingers 240 snap inwardly to move the tooth or teeth 242 into engagement with the teeth 232 to secure the first and second beams 212, 214 in one of the plurality of clamped positions. The ligation clip 200 operates in a substantially similar manner to the ligation clips 10 and 100. Accordingly, a description of the operation of the ligation clip 200 is not provided herein.

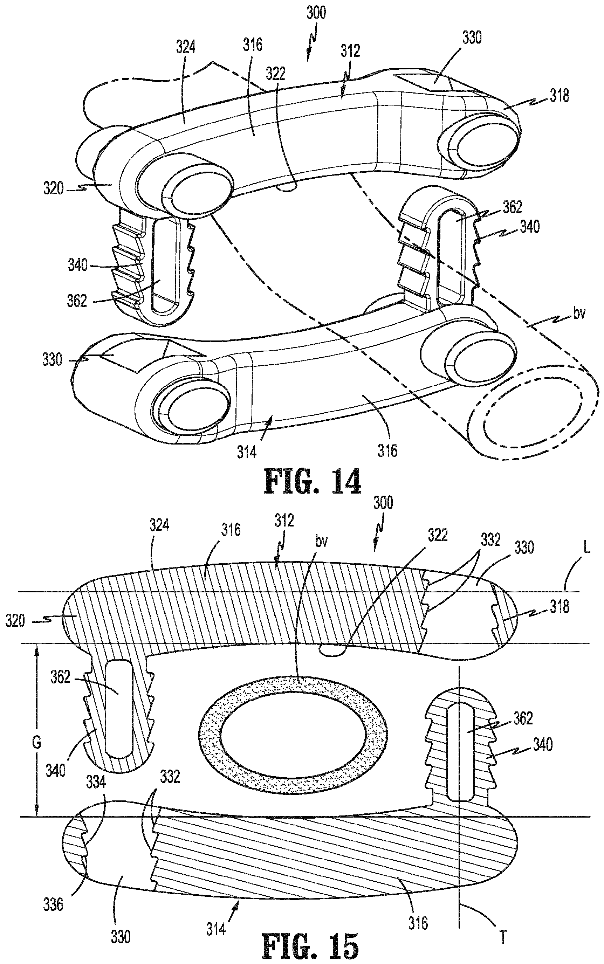

FIGS. 14-16 illustrate other aspects of the disclosed two-piece ligation clip shown generally as ligation clip 300. The ligation clip 300 is substantially identical to the ligation clips 10, 100, and 200 (FIG. 1) but includes a modified latching structure. Accordingly, only the latching structure of the ligation clip 300 will be described in detail in this application.

The ligation clip 300 includes a first piece or beam 312 and a second piece or beam 314. As described above in regard to the ligation clips 10, 100, and 200, the ligation clip 300 can move between open and clamped positions. In the open position illustrated in FIGS. 14 and 15, the first and second beams 312, 314 are spaced from each other to define a gap "G" to receive tissue, e.g., body vessel "bv". In certain aspects of the disclosure, the beams 312, 314 can be mirror images of each other, wherein each of the beams 312, 314 includes a body 316 that defines a longitudinal axis "L" (FIG. 15) and has a first end portion 318, a second end portion 320, an inner surface 322, and an outer surface 324. The first end portion 318 of each of the first and second beams 312, 314 defines a through bore 330 that extends from the inner surface 322 of the body 316 to the outer surface 324 of the body 316. In certain aspects of the disclosure, teeth 332 are spaced along a wall defining the through bore 330. Each of the teeth 132 has a ramped surface 334 and a lock surface 336 that defines a plane that is substantially parallel to the longitudinal axis "L" (FIG. 15) of the respective beam 312, 314.

The second end 320 of the body 316 of each of the beams 312, 314 supports a finger 340. Each of the fingers 340 defines an axis "T" (FIG. 15) that is substantially transverse to the longitudinal axis "L" of the respective beam 312, 314. The fingers 340 and the through bore 330 form the latching structure of the ligation clip 300 and function to retain the ligation clip 300 in one of a plurality of clamped positions about the body vessel "bv". In one aspect of the disclosure, each of the fingers 340 is substantially cylindrical and the through bore 330 is circular. When the first and second beams 312, 314 are moved to one of the plurality of clamped positions, the fingers 340 are received within the through bore 330.

In certain aspects of the disclosure, each of the fingers 340 includes at least one outwardly facing tooth 342 that sequentially engages the teeth 332 located on the wall defining the through bore 330 of a respective beam 312, 314 to secure the first and second beams 312, 314 together about tissue "bv" in several distinct positions. In each of the distinct positions, the gap "G" defined between the beams 312, 314 is different to accommodate tissues of different thicknesses. The at least one tooth 342 also has a ramped surface 344 and a lock surface 346 that defines a plane that is substantially parallel to the longitudinal axes "L" of the first and second beams 312, 314 when the ligation clip 300 is in a clamped position.

When the fingers 340 are inserted into the respective through bores 330 of the respective first and second beams 312, 314 to move the first and second beams 312, 314 to the clamped positions, the fingers 340 engage the wall that defines the through bore 330 and initially flex inwardly to allow the tooth or teeth 342 to pass over the respective teeth 132. As the tooth or teeth 342 pass over each of the respective teeth 332, the fingers 340 snap outwardly to move the tooth or teeth 342 into engagement with the teeth 332 to secure the first and second beams 312, 314 in one of the plurality of clamped positions. In certain aspects of the disclosure, the fingers 340 are hollow and define a cavity 362. The cavity 362 allow the walls of the fingers 340 to flex inwardly to allow the teeth 342 to sequentially pass over the teeth 332 as the first and second beams 312, 314 move to their clamped positions.

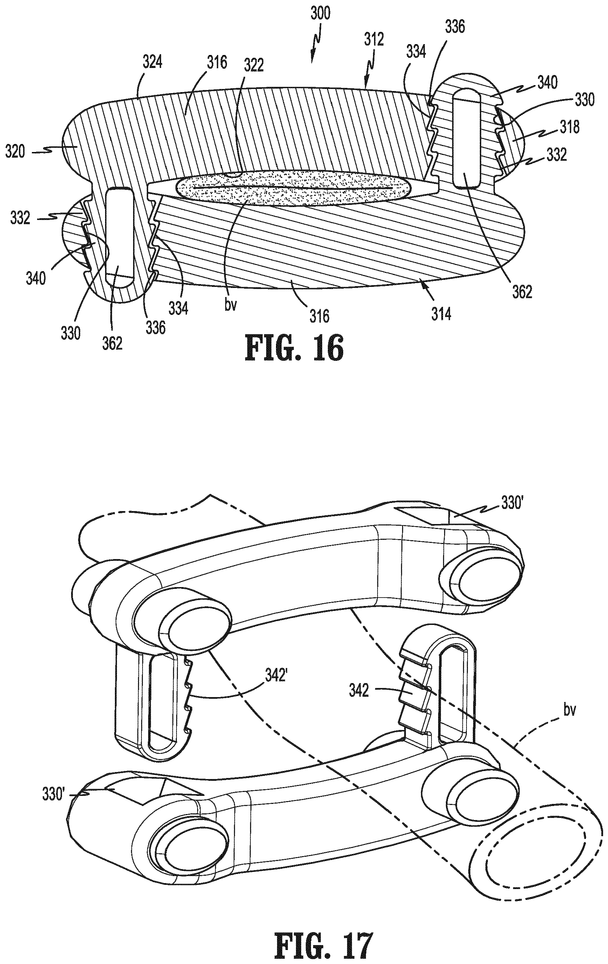

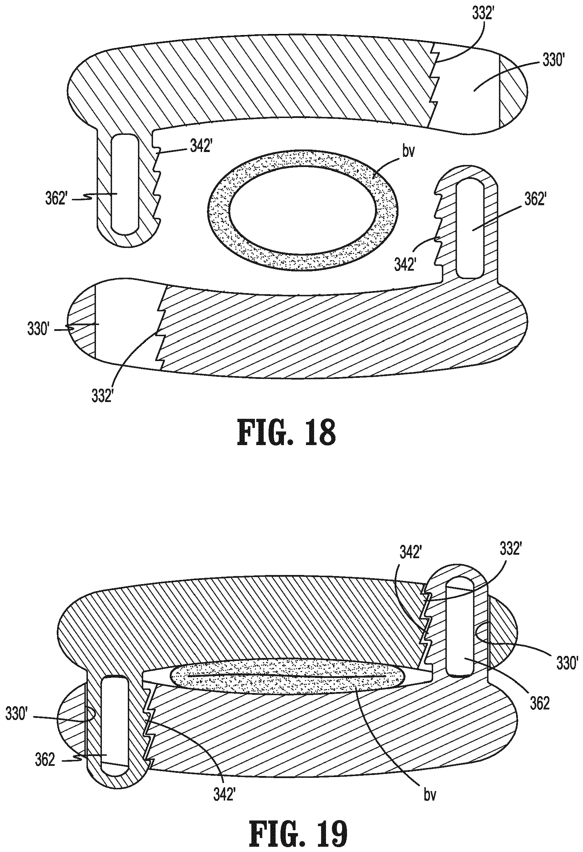

As illustrated in FIGS. 14-16, the teeth 342 can be positioned on opposite sides of the fingers 340 and the teeth 332 can be positioned on opposite walls defining the through bore 330. Alternately, as shown in FIGS. 17-20, the teeth 342' can be positioned on one side of the fingers 340' and the teeth 332' can be positioned on one wall defining the through bore 330'.

The ligation clip 300 operates in a substantially similar manner to the ligation clip 10. Accordingly, a description of the operation of the ligation clip 100 is not provided herein.

Persons skilled in the art will understand that the devices and methods specifically described herein and illustrated in the accompanying drawings are non-limiting exemplary aspects of the disclosed ligation clip. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described aspects of the disclosed ligation clip. Accordingly, the disclosure is not to be limited by what has been particularly shown and described, except as indicated by the appended claims.

The invention may be described by reference to the following numbered paragraphs:
1. A ligation clip comprising: a first beam having a first end portion, a second end portion, and a first clamping surface positioned between the first and second end portions, the first end portion of the first beam having a first finger and a second end portion of the first beam defining a first through bore, the first beam defining a first longitudinal axis; and a second beam having a first end portion, a second end portion, and a second clamping surface positioned between the first and second end portions, the first end portion of the second beam having a second finger and the second end portion of the second beam defining a second through bore, the second beam defining a second longitudinal axis; wherein the first finger of the first beam is received within the second through bore of the second beam and the second finger of the second beam is received within the first through bore of the first beam to secure the ligation clip in a clamped position.
2. The ligation clip of paragraph 1, wherein the first and second fingers include first latching structure and the first and second through bores include second latching structure, the first latching structure being engagable with the second latching structure to retain the ligation clip in the clamped position.
3. The ligation clip of paragraph 2, wherein in the clamped position the first and second clamping surfaces define a gap "G", the first latching structure of the first and second fingers being received within the first and second through bores in a plurality of relative positions to vary the size of the gap "G" of the ligation clip to accommodate tissues of different thicknesses.
4. The ligation clip of paragraph 3, wherein the first latching structure includes at least one first tooth formed on each of the first and second fingers and the second latching structure includes at least one second tooth formed on a wall defining each of the through bores, the at least one first tooth being engageable with the at least one second tooth to retain the ligation clip in one of the clamped positions.
5. The ligation clip of paragraph 4, wherein the at least one first tooth includes a plurality of first teeth.
6. The ligation clip of paragraph 5, wherein the at least one second tooth includes a plurality of second teeth.
7. The ligation clip of paragraph 1, wherein the first end portion of each of the first and second beams includes first posts, the first posts extending radially outward of the first and second clamping surfaces.
8. The ligation clip of paragraph 7, wherein the second end portion of each of the first and second beams includes second posts, the second posts extending radially outward of the first and second clamping surfaces.
9. The ligation clip of paragraph 4, wherein the first and second beams are mirror images of each other.
10. The ligation clip of paragraph 4, wherein each of the at least one first tooth and each of the at least one second tooth includes a ramped surface and a lock surface, the lock surface defining a plane that is substantially parallel to the longitudinal axes of the first and second beams.
11. The ligation clip of paragraph 1, wherein the ramped surfaces of the at least one first tooth and the ramped surface of the at least one second tooth are positioned to engage each other as the ligation clip is moved towards the clamped position such that the at least one first tooth passes over the at least one second tooth in ratcheting fashion.
12. The ligation clip of paragraph 2, wherein the lock surfaces of the at least one first tooth and the at least one second tooth are movable into engagement to retain the ligation clip in the clamped position.
13. The ligation clip of paragraph 2, wherein the first finger defines a first pair of spaced fingers and the second finger defines a second pair of spaced fingers, each of the first and second pairs of spaced fingers defining a channel.
14. The ligation clip of paragraph 13, wherein the first beam includes a first dividing wall positioned within the first through bore and the second beam includes a second dividing wall positioned within the second through bore, wherein the first dividing wall is received within the channel defined by the first pair of spaced fingers and the second dividing wall is received in the channel defined by the second pair of spaced fingers.
15. The ligation clip of paragraph 14, wherein the second latching structure is formed on walls defining the first and second through bores, and the first latching structure is formed on outwardly facing surfaces of the first and second pairs of spaced fingers.
16. The ligation clip of paragraph 14, wherein the second latching structure is formed on the first and second dividing walls, and the first latching structure is formed on inwardly facing surfaces of the first and second pairs of spaced fingers.
17. The ligation clip of paragraph 2, wherein each of the first and second fingers includes a wall defining a cavity, the walls being flexible to allows wall the fingers to flex inwardly.
18. A ligation clip comprising: a first beam having a first end portion, a second end portion, and a first clamping surface positioned between the first and second end portions, the first end portion of the first beam having a first finger and the second end portion of the first beam defining a first through bore, the first beam defining a first longitudinal axis; and a second beam having a first end portion, a second end portion, and a second clamping surface positioned between the first and second end portions, the first end portion of the second beam having a second finger and the second end portion of the second beam defining a second through bore, the first finger of the first beam being received within the second through bore of the second beam and the second finger of the second beam being received within the first through bore of the first beam to secure the ligation clip in a clamped position, the second beam defining a second longitudinal axis; wherein the first beam is a mirror image of the second beam.
19. A ligation clip comprising: a first beam having a first end portion, a second end portion, and a first clamping surface positioned between the first and second end portions, the first end portion of the first beam having a first finger and a second end portion of the first beam defining a first through bore, the first beam defining a first longitudinal axis; and a second beam having a first end portion, a second end portion, and a second clamping surface positioned between the first and second end portions, the first end portion of the second beam having a second finger and the second end portion of the second beam defining a second through bore, the second beam defining a second longitudinal axis; wherein the first and second fingers include a plurality of first teeth and the first and second through bores include a plurality of second teeth, the plurality of first teeth being engageable with the plurality of second teeth to retain the ligation clip in the clamped position, the first finger of the first beam being received within the second through bore of the second beam and the second finger of the second beam being received within the first through bore of the first beam to secure the ligation clip in a clamped position.
20. The ligation clip of paragraph 19, wherein the first finger defines a first pair of spaced fingers and the second finger defines a second pair of spaced fingers, each of the first and second pairs of spaced fingers defining a channel.

## Claims

1. A ligation clip comprising:
a first beam having a first end portion, a second end portion, and a first clamping surface positioned between the first and second end portions, the first end portion of the first beam having a first finger and a second end portion of the first beam defining a first through bore, the first beam defining a first longitudinal axis; and
a second beam having a first end portion, a second end portion, and a second clamping surface positioned between the first and second end portions, the first end portion of the second beam having a second finger and the second end portion of the second beam defining a second through bore, the second beam defining a second longitudinal axis;
wherein the first finger of the first beam is received within the second through bore of the second beam and the second finger of the second beam is received within the first through bore of the first beam to secure the ligation clip in a clamped position.

2. The ligation clip of claim 1, wherein the first and second fingers include first latching structure and the first and second through bores include second latching structure, the first latching structure being engagable with the second latching structure to retain the ligation clip in the clamped position.

3. The ligation clip of claim 2, wherein in the clamped position the first and second clamping surfaces define a gap "G", the first latching structure of the first and second fingers being received within the first and second through bores in a plurality of relative positions to vary the size of the gap "G" of the ligation clip to accommodate tissues of different thicknesses; preferably wherein the first latching structure includes at least one first tooth formed on each of the first and second fingers and the second latching structure includes at least one second tooth formed on a wall defining each of the through bores, the at least one first tooth being engageable with the at least one second tooth to retain the ligation clip in one of the clamped positions.

4. The ligation clip of claim 3, wherein the at least one first tooth includes a plurality of first teeth; preferably wherein the at least one second tooth includes a plurality of second teeth.

5. The ligation clip of any preceding claim, wherein the first end portion of each of the first and second beams includes first posts, the first posts extending radially outward of the first and second clamping surfaces; preferably wherein the second end portion of each of the first and second beams includes second posts, the second posts extending radially outward of the first and second clamping surfaces.

6. The ligation clip of any of claims 3 to 5, wherein the first and second beams are mirror images of each other, and/or wherein each of the at least one first tooth and each of the at least one second tooth includes a ramped surface and a lock surface, the lock surface defining a plane that is substantially parallel to the longitudinal axes of the first and second beams.

7. The ligation clip of any preceding claim, wherein the ramped surfaces of the at least one first tooth and the ramped surface of the at least one second tooth are positioned to engage each other as the ligation clip is moved towards the clamped position such that the at least one first tooth passes over the at least one second tooth in ratcheting fashion.

8. The ligation clip of claim 2, wherein the lock surfaces of the at least one first tooth and the at least one second tooth are movable into engagement to retain the ligation clip in the clamped position; and/or wherein the first finger defines a first pair of spaced fingers and the second finger defines a second pair of spaced fingers, each of the first and second pairs of spaced fingers defining a channel.

9. The ligation clip of claim 8, wherein the first beam includes a first dividing wall positioned within the first through bore and the second beam includes a second dividing wall positioned within the second through bore, wherein the first dividing wall is received within the channel defined by the first pair of spaced fingers and the second dividing wall is received in the channel defined by the second pair of spaced fingers.

10. The ligation clip of claim 9, wherein the second latching structure is formed on walls defining the first and second through bores, and the first latching structure is formed on outwardly facing surfaces of the first and second pairs of spaced fingers.

11. The ligation clip of claim 9, wherein the second latching structure is formed on the first and second dividing walls, and the first latching structure is formed on inwardly facing surfaces of the first and second pairs of spaced fingers.

12. The ligation clip of claim 2, wherein each of the first and second fingers includes a wall defining a cavity, the walls being flexible to allows wall the fingers to flex inwardly.

13. A ligation clip comprising:
a first beam having a first end portion, a second end portion, and a first clamping surface positioned between the first and second end portions, the first end portion of the first beam having a first finger and the second end portion of the first beam defining a first through bore, the first beam defining a first longitudinal axis; and
a second beam having a first end portion, a second end portion, and a second clamping surface positioned between the first and second end portions, the first end portion of the second beam having a second finger and the second end portion of the second beam defining a second through bore, the first finger of the first beam being received within the second through bore of the second beam and the second finger of the second beam being received within the first through bore of the first beam to secure the ligation clip in a clamped position, the second beam defining a second longitudinal axis;
wherein the first beam is a mirror image of the second beam.

14. A ligation clip comprising:
a first beam having a first end portion, a second end portion, and a first clamping surface positioned between the first and second end portions, the first end portion of the first beam having a first finger and a second end portion of the first beam defining a first through bore, the first beam defining a first longitudinal axis; and
a second beam having a first end portion, a second end portion, and a second clamping surface positioned between the first and second end portions, the first end portion of the second beam having a second finger and the second end portion of the second beam defining a second through bore, the second beam defining a second longitudinal axis;
wherein the first and second fingers include a plurality of first teeth and the first and second through bores include a plurality of second teeth, the plurality of first teeth being engageable with the plurality of second teeth to retain the ligation clip in the clamped position, the first finger of the first beam being received within the second through bore of the second beam and the second finger of the second beam being received within the first through bore of the first beam to secure the ligation clip in a clamped position.

15. The ligation clip of claim 14, wherein the first finger defines a first pair of spaced fingers and the second finger defines a second pair of spaced fingers, each of the first and second pairs of spaced fingers defining a channel.
